# EUROPEAN PATENT APPLICATION

(11) **EP 4 410 762 A1**
(43) Date of publication of application: **07.08.2024**
(21) Application number: 23155183.9
(22) Date of filing: 06.02.2023
(51) Int. Cl.: C07C 1/30, C07C 17/154, C07C 17/38, C07C 17/383, C25B 1/24

(54) **PROCESS AND PLANT FOR PRODUCING PROPYLENE AND HYDROGEN FROM PROPANE**

(71) Applicant: Sulzer Management AG, 8401 Winterthur (CH)
(72) Inventor: The designation of the inventor has not yet been filed
(74) Representative: Henkel & Partner mbB

(57) **Abstract**

A process for producing propylene and hydrogen from propane comprises the following steps:
a) reacting a mixture comprising propane and bromine so as to produce a first reaction mixture containing bromopropane and hydrogen bromide,
b) subjecting the first reaction mixture obtained in step a) to at least one separation step so as to obtain a bromopropane enriched composition and to obtain a hydrogen bromide enriched composition,
c) catalytically reacting the bromopropane enriched composition obtained in step b) so as to produce a second reaction mixture containing propylene and hydrogen bromide,
d) subjecting the second reaction mixture obtained in step c) to at least one separation step so as to obtain a hydrogen bromide enriched composition and to obtain propylene and
e) subjecting the hydrogen bromide enriched composition obtained in step b) and/or the hydrogen bromide enriched composition obtained in step d) to an electrolysis so as to obtain hydrogen and a bromine containing composition, wherein preferably at least a portion of the bromine containing composition is recycled back into step a).

## Description

The present invention relates to a process and to a plant for producing propylene and hydrogen from propane.

Propane is a common product of a refinery or of a natural processing plant. Currently, propane is either valorized on account of its calorific value or used as starting material for the production of propylene. During the current period of the energy transition many refineries look for options to boost petrochemicals production based on propane feedstock. However, the amount of the available propane from a single refinery is relatively limited and cannot economically justify the investment in on-purpose propylene production via a direct propane dehydrogenation. Therefore, in most cases the propane is typically converted in a steam cracker to a mixture comprising about 28 % of fuel gases, which are burnt resulting in a significant carbon dioxide emission. The yield of fuel gases resulting from the conversion in a steam cracker of propane is much higher in comparison with that obtained from ethane feedstock, namely about 8 % fuel gas, or from naphtha feedstock, namely about 12 to 15%. In view of this, propane is very inefficient and a polluting feedstock for a steam cracker. Thus, there is a need for new technologies to valorize propane feedstocks.

In recent years, a new biosourced propane has emerged, which offers an opportunity to produce biopropylene. However, once again an efficient propane-to-propylene technology, which is economically attractive even at a small-mid scale, is necessary. Currently, there are several known biomass-to-energy conversion processes, in which biopropane is produced as a co-product, wherein a plethora of biomass feedstocks can be used in those processes. These processes range from the hydrotreating of vegetable oil so as to produce hydrotreated vegetable oil (HVO) biodiesel to the production of synthetic fuels through gasification and synthesis. Presently, the primary production route for biopropane is as a co-product of HVO biodiesel, wherein the process yield lies in the range of 5 to 8%. Due to the large amounts of HVO biodiesel produced, significant volumes of biopropane can be obtained. There is also a small number of novel processes, with which biopropane can be produced as the main target product. These include the use of specialized microbes to ferment organic wastes so as to produce biopropane (rather than biomethane), and also the direct synthesis of propane from the syngas produced from the gasification of woody biomass.

Propylene is a particular valuable raw material for chemical syntheses and in fact, together with ethylene, one of the two most important starting materials in the petrochemical industry. For instance, propylene is the starting material for producing polypropylene, which is one of the most widely used polymers, since it is odorless, skin-friendly, physiologically harmless and has excellent mechanical properties. For example, polypropylene is used for packings, films, textiles, caps, medical products, mattress ticks and many other articles. Furthermore, propylene is used for the productions of other important chemicals, such as for producing propylene glycol, acrylonitrile, acrylic acid and propylene oxide.

Also hydrogen is an important compound, which is of particular importance as ecological sustainable energy source, because it produces only water, when used as energy source of a fuel cell or if burned with oxygen. Moreover, hydrogen is an important raw material for the production of fertilizers as well as for the refinement of mineral oil and is used as cooling agent for power stations and many other purposes. On account of its ecological sustainable properties, it is expected that the global demand for hydrogen and hydrogen-derived fuels will significantly increase in the coming years, particularly because it is expected that hydrogen will play a key role in mitigating the global warming.

Hydrogen is commonly produced by electrolysis of water, such as by alkaline electrolysis, proton exchange membrane electrolysis or solid oxide electrolysis. The different electrolysis technologies have different operation ranges and different efficiencies. While solid oxide electrolysis is for example a high temperature solution that typically offers comparably high efficiencies, this technique has drastic drawbacks, such as having a limited lifetime and requiring relatively high capital expenditures in combination with generally lower technology readiness levels. Alkaline electrolysis does not allow to regulate power over a comparably broad range and furthermore suffers from disadvantageously low current densities, which in turn increase the capital expenditures. In contrast thereto, proton exchange membrane electrolysis can be typically operated at higher current densities, which reduces the capital expenditures. Moreover, proton exchange membrane electrolysis is much more flexible than other water electrolysis techniques, such as in particular alkaline electrolysis. In view of this, proton exchange membrane electrolysis is generally favored for water electrolysis in order to produce green hydrogen. However, water electrolysis requires a significant amount of energy. Therefore, alternative electrolysis routes to produce hydrogen would be desirable.

In turn, propylene is presently mainly produced in steam crackers and refineries and a few on-purpose technologies are also known. The production of this petrochemicals is accompanied with a significant carbon dioxide emission, thus new low carbon technologies are necessary in order to improve the situation. More specifically, propylene is commonly produced by a direct catalytic dehydrogenation of propane to propylene, which is performed at a relatively high temperature of 550 to 680°C and at a comparable low-pressure, which is typically below atmospheric pressure. However, such processes based on the direct catalytic dehydrogenation of propane are - among others on account of the required high reaction temperature - energy intensive and ecologically harmful, if non-regenerable fuels, such as coal or the like, forming tremendous amounts of carbon dioxide during their combustion, are used to generate the required energy for maintaining the required high reaction temperature. The predominantly used direct dehydrogenation processes utilize circulating catalysts or multiple reactors circulating in sequence production-purge-regeneration-purge. This requirement derives from the propane-propylene equilibrium limitation, which is mitigated by the high temperature and/or low-pressure operation. All in all, these processes based on the direct catalytic dehydrogenation of propane have the drawback of high capital expenditures, of high operational costs and of producing high amounts of carbon dioxide. These types of process are, if at all, only economical for a mega-scale, such as with propylene production capacities above 500 kt/y. In contrast, a conventional propane dehydrogenation is not suitable to boost the petrochemical production at a refinery, where the availability of a significant amount of propane should be guaranteed for a very long period.

Other processes for producing propylene are known, such as for instance processes based on a chemical looping. For example, a process for producing propylene is known, in which a bromopropane is reacted with copper oxide to form propylene, copper bromide and water, before the copper oxide is regenerated by reacting copper bromide with oxygen to bromine and copper oxide. However, this process is very energy intensive. Also known are processes, in which propane is brominated with bromine and the so obtained bromopropane is dehydrobrominated to propylene and hydrogen bromide, before bromine is recovered from the hydrogen bromide by oxidizing hydrogen bromide to bromine and water. However, this process requires the separation of the bromine from the water, which is very energy intensive so that such processes are characterized by high operational costs.

All in all, the known processes for producing propylene suffer from at least one and most of them from two or more of the disadvantages of high operational costs, of producing high amounts of carbon dioxide and of requiring high capital expenditures. Moreover, none of these processes allows to also produce on-purpose the valuable hydrogen electrochemically. However, the electrochemical production of hydrogen would allow to also utilize renewable electricity. In turn, the known processes for producing hydrogen are energy intensive and do not allow to also produce propylene.

In view of this, the object underlying the present invention is to provide a process for producing both, propylene and hydrogen, wherein the process can be operated in an industrial scale, has a comparable low energy demand, is ecologically friendly and in particular does not produce high amounts of carbon dioxide and does not require high capital expenditures.

In accordance with the present invention, this object is solved by providing a process for producing propylene and hydrogen from propane comprising the following steps:
a) reacting a mixture comprising propane and bromine so as to produce a first reaction mixture containing bromopropane and hydrogen bromide,
b) subjecting the first reaction mixture obtained in step a) to at least one separation step so as to obtain a bromopropane enriched composition and to obtain a hydrogen bromide enriched composition,
c) catalytically reacting the bromopropane enriched composition obtained in step b) so as to produce a second reaction mixture containing propylene and hydrogen bromide,
d) subjecting the second reaction mixture obtained in step c) to at least one separation step so as to obtain a hydrogen bromide enriched composition and to obtain propylene and
e) subjecting the hydrogen bromide enriched composition obtained in step b) and/or the hydrogen bromide enriched composition obtained in step d) to an electrolysis so as to obtain hydrogen and a bromine containing composition.

Thereby, hydrogen and propylene may be produced in an ecologically friendly manner with comparable low operational costs and comparable low capital expenditures. Moreover, the process in accordance with the present invention is characterized by a high propylene yield. Since the process in accordance with the present invention does not base on a direct conversion of propane to propylene, the process is - in contrast to the known processes of directly catalytically dehydrogenating propane to propylene - not limited by the propane-propylene equilibrium, why a reaction at comparable high temperatures is not necessary. On the contrary, the dehydrobromination reaction of step c) may be performed at a comparable low reaction temperature. Thereby not only the operational costs decreases, but also the propylene yield increases and a disadvantageous coking due to high temperatures is reliably avoided. Moreover, since the reaction in step c) is performed catalytically, an energy intensive chemical looping, as used in some of the prior art processes, can be avoided. In addition, the regeneration of bromine from the hydrogen bromide in step e) by electrolysis positively contributes to the low operational costs and low capital expenditures of the process according to the present invention, because an energy intensive separation of water and hydrogen bromide from a mixture containing both is not required. Moreover, the process making use of an electrolysis also allows to produce pure hydrogen. All in all, the process in accordance with the present invention produces both, propylene and hydrogen, can be operated in an industrial scale, has a comparable low energy demand, is ecologically friendly and in particular does not produce high amounts of carbon dioxide and does not require high capital expenditures.

In step a) a mixture comprising propane and bromine is reacted so as to produce a first reaction mixture containing bromopropane and hydrogen bromide. Good results are in particular obtained, when the mixture comprising propane and bromine used in step a) contains 10 to 90% by volume and preferably 50 to 80% by volume of propane as well as 10 to 50% by volume and preferably 20 to 45% by volume of bromine. Moreover, it is preferred that the mixture comprises less than 0.5% by volume of unsaturated compounds, such as in particular propylene, unsaturated hydrocarbon compounds and unsaturated brominated hydrocarbon compounds. This allows to limit the polybrominated compounds in the first reaction mixture and to easily separate non-reacted propane from the reaction mixture and to recycle the propane into the mixture fed into the bromination reaction a).

The process in accordance with the present invention is not particularly limited concerning the source of the propane contained in the mixture being reacted in step a). For instance, pure propane may be used and admixed with bromine so as to obtain the mixture used in step a). However, it is also possible to use a crude composition including propane as one of several ingredients. Suitable examples therefore are natural gas, refinery gas and petroleum gas. Therefore, it is possible to react in step a) a mixture, which contains a gas being selected from the group consisting of natural gas, refinery gas, petroleum gas, biopropane produced at biodiesel plant, biopropane synthesized from glycerol, biopropane produced by fermentation of organic waste as biogas, biopropane being directly synthesized from the syngas produced from the gasification of woody biomass or other renewable sources and arbitrary combinations of two or more of the aforementioned gases and which further contains bromine. The mixture may optionally further contain purified propane and actually preferably contains purified propane, which is separated from the first reaction mixture and recycled into the mixture being reacted in step a).

Propane feedstock often contains substantial amounts of propylene. Moreover, in some embodiments of the present invention, during the reaction of step a) also minor amounts of propylene are formed and thus contained in the first reaction mixture, which may be, as set out further below, recycled together with a purified propane composition into step a). In such cases, it is preferred that the propane feedstock before being added into the mixture being reacted in step a) or the mixture being reacted in step a) is subjected to at least one purification step in order to remove selectively propylene from the propane feedstock or mixture being reacted in step a), respectively. The process in accordance with the present invention is not particularly limited concerning the method for purifying the propane enriched composition. For instance, the currently commercially practiced approaches, namely distillation and catalytic hydrogenation, may be used. Distillation is a viable technology for the removal of propylene from propane, but both, the capital as well as the energy costs are high due to the very low relative volatility between the two species. Catalytic hydrogenation is straightforward, but also has a high capital cost, and the cost/availability of the continuous hydrogen production required may also be problematic. Alternative approaches are based on absorption/stripping based on aqueous silver nitrate solution and pressure-swing adsorption (PSA) based on zeolite molecular sieves, preferably silver containing molecular sieves, i.e AgX zeolite. Membranes may also be used, namely in a single stage separation, and leads with simple, compact, and robust equipment to separation results of propylene-propane being equivalent to a distillation comprising many stages. Chemically selective sorbents offer even higher degrees of separation in a single stage, albeit requiring more complex equipment. An interesting option is the selective oxidation of propylene to carbon monoxide on a silver-containing catalyst. If the concentration of propylene in the composition to be purified is very high, such as 10 % by weight or higher, only distillation or direct hydrogenation are preferred. If the concentration of propylene in the composition to be purified is in a middle range of 2 to 8% by weight, membranes and physical adsorption are promising options. If the concentration of propylene in the composition to be purified is it is quite low, i.e. less than 2% by weight, chemical absorption and adsorption are most preferred separation techniques. If the concentration of propylene in the composition to be purified is less than 2% by weight, distillation and membranes are not economical and if the concentration of propylene in the composition to be purified is 0.1% by weight or less, only chemical absorption, adsorption or hydrogenation is preferred. The principle of absorptive separation bases on the contact of a propane-propylene mixture with a liquid containing, such as for example silver(I)ions. The propylene binds to the silver, while the propane does not. Thus, the propane loading is limited to its physical solubility, while the propylene loading can approach up to a 0.3:1 mole ratio with the silver loading. The liquid is then depressurized or heated to break the propylene-silver bonds, and relatively pure propylene is recovered. In its simplest implementation, the system looks and works much like an amine absorber/stripper for removal of acid gases. In turn, the principle of adsorptive separation of propane/propylene by PSA for may be based, for example, on 13X zeolites. Alternatively, the Petrofin process may be used, which utilizes a 4A zeolite and a process specifically tailored for "kinetic" propylene/propane separation. Kinetic separation is possible when two competing species are sorbed roughly equally at equilibrium, but one is sorbed "faster" than the other, due to higher diffusion rates in the pores. Such is the case of the Petrofin process, in which the propylene diffuses into the pores much more quickly than the propane does, so that by keeping the PSA cycle times far too low to allow true equilibrium to be attained, propylene can be selectively removed.

The present invention is not particularly restricted concerning the ratio of the propane and bromine in the mixture used in step a). Good results are in particular obtained, when the molar ratio of propane to bromine in the mixture reacted used in step a) is 10:1 to 1:1 and preferably 3:1 to 2:1.

The reaction in step a) may be performed in the presence of a catalyst. However, in order to save costs, it is proposed in a further development of the idea of the present invention to only thermally induce the reaction in step a) and to react the mixture in step a) without catalyst.

In order to thermally induce the reaction in step a), the mixture is reacted in step a) preferably at a temperature of 200 to less than 420°C, more preferably at a temperature of 230 to 410°C, still more preferably at a temperature of 240 to 400°C and most preferably at a temperature of 240 to 300°C. Apart from being high enough so as to thermally induce the reaction in step a), the aforementioned temperatures are low enough so as to minimize the undesired formation of unsaturated compounds, such as propylene and allyl bromide, as well as the undesired formation of coke in step a). This is advantageous, because allyl bromide has a boiling temperature being very close to that bromopropane and is thus very difficult to separate from the bromopropane for instance by distillation. Furthermore, allyl bromide may lead to a deactivation of the dehydrobromination catalyst used in step d). The avoidance of propylene formation is advantageous, since otherwise non-reacted propane being containing in the first reaction mixture could not been efficiently separated at low operational costs from the first reaction mixture and recycled into step a).

Preferably, the mixture is reacted in step a) at an elevated pressure, more preferably at a pressure of at least 400 kPa, still more preferably at a pressure of at least 1 MPa and most preferably at a pressure of 1 to 4 MPa. The elevated pressure leads to a high conversion rate per time unit and advantageously avoids or at least minimizes the formation of unsaturated products, such as of allyl bromide or propylene, in the reaction mixture.

In accordance with a further particular preferred embodiment of the present invention, the residence time of the mixture in step a) is at least 10 seconds and more preferably 20 seconds to 2 minutes. Residence time means in this connection the time period between the entrance of the mixture into the reactor, in which the reaction is performed, and the exit of the first reaction mixture from the reactor. A residence time of at least 210 seconds assures that all or at least essentially all of the bromine is consumed. This is important, because the quite reactive bromine would require, if being contained in a certain amount in the reaction mixture, to use downstream of the reactor vessels made of more expensive materials in order to make them resistant to bromine.

The reaction in step a) may be performed in any reactor type, such as in isothermal reactors or adiabatic reactors. For instance, the mixture is reacted in step a) in an adiabatic reactor, preferably with an outlet temperature of less than 450°C, more preferably an outlet temperature of at most 420°C and still more preferably an outlet temperature of at most 400°C.

In practice, not all of the bromine and/or propane will react during the reaction in step a) to bromopropane, but also polybromopropanes will be formed and small amounts of bromine and propane will remain as non-reacted educts in the reaction mixture. Therefore, the first reaction mixture obtained in step a) will in practice further contain one or more polybromopropanes, non-reacted propane and optionally at least traces of non-reacted bromine. Polybromopropane means any brominated propane having two or more bromine residues per molecule, i.e. any dibromopropane, tribromopropane or higher bromopropane.

In step b) of the process in accordance with the present invention, the first reaction mixture obtained in step a) is subjected to at least one separation step so as to obtain a bromopropane enriched composition and to obtain a hydrogen bromide enriched composition. Bromopropane enriched composition means any composition having after the separation step(s) of step b) a higher bromopropane concentration than the first reaction mixture. Likewise, hydrogen bromide enriched composition means any composition having after the separation step(s) b) a higher hydrogen bromide concentration than the first reaction mixture. Preferably, the bromopropane enriched composition has a bromopropane concentration of at least 10% by weight and more preferably of at least 50% by weight.

Preferably, step b) comprises at least one and preferably two distillation steps for separating bromopropane from the first reaction mixture so as to obtain the bromopropane enriched composition.

Furthermore, it is preferred that step b) comprises at least one absorption step for separating hydrogen bromide from the first reaction mixture so as to obtain the hydrogen bromide enriched composition. Good results are in particular obtained, when in the at least one absorption step water or an aqueous composition is used as absorbing agent. For instance, the absorbing agent may be an aqueous composition containing at least 80% by weight and preferably at least 95% by weight of water and reminder to 100% by weight one or more compounds, such as, for instance, hydrogen bromide. It is particularly preferred that the aqueous composition used as absorbing agent contains hydrogen bromide, such as hydrogen bromide resulting from the recovery of the used absorbing agent, which is preferably used in circuit, i.e. recovered after the absorption step.

In addition, it is preferred that the hydrogen bromide enriched composition has a hydrogen bromide concentration of at least 30% by weight and more preferably of at least 40% by weight as well as a content of absorbing agent of at most 60% by weight and more preferably of at most 48% by weight. If the absorbing agent is water, in fact the hydrogen bromide enriched composition is an aqueous solution containing hydrogen bromide.

In accordance with a particular preferred embodiment of the present invention, step b) comprises a distillation step for distilling the first reaction mixture so as to obtain a bromopropane and polybromopropane enriched composition as bottom composition and an overheads composition containing hydrogen bromide, non-reacted propane and non-reacted bromine. The overheads composition obtained in the distillation step is then preferably subjected to an absorption step with water or an aqueous composition so as to obtain a purified propane composition (or propane enriched composition, respectively) and to obtain the hydrogen bromide enriched composition being a mixture comprising hydrogen bromide, water and optionally traces of bromine. The purified propane composition may be dried afterwards, in order to remove traces of water being still contained therein. The obtained purified propane composition contains preferably at least 80% by weight and more preferably at least 95% by weight of propane as well as less than 500 ppm and more preferably less than 100 ppm of water and other impurities. At least a portion, such as at least 80 % by volume, and preferably all of the purified propane composition is recycled to the mixture used in step a). Recycling the purified propane composition into step a) means that the purified propane composition is mixed with a propane containing starting composition, such as natural gas, and with bromine so as to form the mixture being reacted in step a).

It is further preferred in this embodiment that the bottom composition obtained in the distillation step is subjected to a (further) distillation step so as to obtain as overheads composition the bromopropane enriched composition and as bottom composition a polybromopropane enriched composition. Particularly preferably, the bromopropane enriched composition, which is in step c) subjected to the dehydrobromination reaction, contains less than 2% by weight of polybromopropane and most preferably less than 0.5% by weight.

The dehydrobromination reaction of step c) is an endothermic reaction and is thus preferably performed at an elevated temperature. Good results are in particular obtained, when the bromopropane enriched composition obtained in step b) is reacted in step c) at a temperature of 250 to 450°C. For instance, in step c) the weight hourly space velocity of the bromopropane in the bromopropane enriched composition is 0.1 to 20 1/h and the partial pressure of the bromopropanes is below 500 kPa and more preferably below 200 kPa.

The dehydrobromination reaction of step c) may be performed in one reactor or in several reactors being arranged in series with an interstage reheating. The reactor or, if two or more reactors are used, each of the reactors may be a fixed bed, a multitubular reactor, a moving bed reactor or a fluidized bed reactor. For example, the dehydrobromination reaction of step c) may be performed in a series of adiabatic fixed bed reactors with an interstage reheating. The fixed bed reactor can also be a radial reactor or a spherical reactor.

In accordance with the present invention, the dehydrobromination reaction of step c) is performed as catalytical reaction. Good results are in particular obtained, when the bromopropane enriched composition obtained in step b) is reacted in step c) in the presence of a catalyst, which is selected from the group consisting of alumina, silica, silicon oxide-aluminium oxides, zeolites with a Si/AI ratio above 25, titanium oxides, zirconium oxides and arbitrary combinations of two or more of the aforementioned materials.

Particularly preferably, the catalyst may in addition contain 0.1 to 10% by weight of at least one transition metal being selected from the group of iron, cobalt, molybdenum, nickel, wolfram, copper, silver, manganese and arbitrary combinations of two or more of the aforementioned metals. The metal containing catalyst may additionally be doped with an element being selected from the group consisting of boron, phosphorous, platinum, palladium and arbitrary combinations of two or more of the aforementioned elements.

It is particularly preferred, if the alumina is gamma-alumina with a high purity. Alumina with a purity of 99.99% or higher may be synthesized for example by the alkoxide route. Alkoxide route is a well-established mass-production technology, which is based on the hydrolysis of aluminum alkoxide. In this process, the alcohol(s) of the aluminum alkoxide is/are recycled back to the synthesis. The alumina is produced either as co-product with synthetic linear alcohols (Ziegler method) or directly from aluminum metal (on-purpose route). The Ziegler route for the synthesis of alumina involves the oligomerization of ethylene using triethylaluminium followed by oxidation. The triethylaluminium may be produced by reacting aluminium, ethylene and hydrogen gas. In the production process, two-thirds of the triethylaluminium produced is recycled back into the reactor and only one-third is used to produce the alcohol. The recycling step is used to produce triethylaluminium at a higher yield and in less time. Triethylaluminium reacts with ethylene to form higher molecular weight trialkylaluminium. The number of equivalents of ethylene n equals the total number of monomer units being grown on the initial ethylene chains, wherein n = x + y + z, wherein x, y, and z are the number of ethylene units per chain. Trialkylaluminium is oxidized with air to form aluminum alkoxides, and finally hydrolyzed to aluminum hydroxide and the desired alcohols.
1. AI+3 C₂H₄ +1.5 H₂→ Al(C₂H₅)₃
2. Al(C₂H₅)₃ + n ethylene → Al((CH₂CH₂)ₙCH₂CH₃)₃
3. Al((CH₂CH₂)ₙCH₂CH₃)₃+ O₂ → Al(O(CH₂CH₂)ₙCH₂CH₃)₃
4. Al(O(CH₂CH₂)ₙCH₂CH₃)₃ → Al(OH)₃ + CH₃CH₂(CH₂C₂)ₘOH

Optionally, the catalyst contains contain 0.1 to 5% by weight of magnesium, calcium, cerium, or lanthanum. For example, the catalyst may be made from magnesium-doped aluminium oxide, calcium doped aluminium oxide, cerium doped aluminium oxide, lanthanum-doped aluminium oxide.

The catalyst may contain a medium pore zeolite with a Si/AI ratio of more than 25 having a pore size of 3.8 to 6 Å (Angstrom). Most preferably, the catalyst is made from medium pore zeolite with a Si/AI ratio of more than 150 having a pore size of 4.5 to 5.5 Å. The zeolite may have been subjected to a steam treatment, to an ion exchange or to a modification with phosphorous before being used as catalyst.

In a further development of the idea of the present invention, it is suggested that the bromopropane enriched composition obtained in step b) is reacted in step c) in the presence of hydrogen. The presence of hydrogen improves the catalyst stability and also prevents a rebromination of the produced propylene. Good results are in particular obtained, when the molar ratio of bromopropane and hydrogen in the mixture being reacted in step c) is 10:1 to 1:10 and more preferably 5:1 to 1:5.

Alternatively, the dehydrobromination reaction of step c) may be performed without the presence of hydrogen, but hydrogen is added after termination of the reaction to the second reaction mixture obtained in step c).

In still a further preferred embodiment of the present invention, a heat vector is added to the reactor, in which the reaction of step c) is performed, so as to make the reaction more isothermal. Suitable examples for heat vectors are nitrogen, argon, C₅₋₁₂-paraffins, water, hydrogen bromide solution and hydrogen bromide gas.

In a further development of the idea of the present invention, it is suggested to recycle a portion of the second reaction mixture into step c) as heat vector.

In accordance with the present invention, the second reaction mixture obtained in step c) is subjected in step d) to at least one separation step so as to obtain a hydrogen bromide enriched composition and to obtain propylene. Again, hydrogen bromide enriched composition means any composition having after the separation step(s) d) a higher hydrogen bromide concentration than the second reaction mixture. Preferably, the propylene contains less than 1,000 ppm and more preferably less than 50 ppm of impurities.

In accordance with a particular preferred embodiment of the present invention, step d) comprises at least one and preferably two purification steps, which are most preferably distillation steps, for separating propylene from the second reaction mixture so as to obtain propylene.

Furthermore, it is preferred that step d) comprises at least one absorption step for separating hydrogen bromide from the second reaction mixture so as to obtain the hydrogen bromide enriched composition. Good results are in particular obtained, when in the at least one absorption step water or an aqueous composition is used as absorbing agent. As in step b), the absorbing agent may contain at least 80% by weight and preferably at least 95% by weight of water and reminder to 100% by weight one or more compounds, such as, for instance, hydrogen bromide. It is particularly preferred that the aqueous composition used as absorbing agent contains hydrogen bromide, such as hydrogen bromide resulting from the recovery of the used absorbing agent, which is preferably used in circuit, i.e. recovered after the absorption step.

In addition, it is preferred that the hydrogen bromide enriched composition has a hydrogen bromide concentration of at least 30% by weight and more preferably of at least 40% by weight as well as a content of absorbing agent of at most 60% by weight and more preferably of at most 48% by weight. Again, if the absorbing agent is water, in fact the hydrogen bromide enriched composition is an aqueous solution containing hydrogen bromide.

In accordance with a particular preferred embodiment of the present invention, step d) comprises a distillation step for distilling the second reaction mixture so as to obtain a polybromopropane enriched composition as bottom composition and an overheads composition containing propylene, hydrogen bromide and optionally hydrogen. The overheads composition obtained in the distillation step is then preferably subjected to an absorption step with water or an aqueous composition so as to obtain propylene or a composition comprising propylene and hydrogen and to obtain the hydrogen bromide enriched composition being a mixture comprising water and hydrogen bromide. If a composition comprising propylene as well as hydrogen is obtained, the hydrogen is then preferably separated from the composition, for example by a pressure swing adsorption, so as to obtain the (pure) propylene.

It is further preferred in this embodiment that the bottom composition obtained in the distillation step is subjected to a (further) distillation step so as to obtain as overheads composition a C₄₋₅-hydrocarbon composition and as bottom composition a polybromopropane enriched composition.

In accordance with the present invention, in step e) the hydrogen bromide enriched composition obtained in step b) and/or the hydrogen bromide enriched composition obtained in step d) is subjected to an electrolysis so as to obtain hydrogen and a bromine containing composition. Preferably, both, the hydrogen bromide enriched composition obtained in step b) as well as the hydrogen bromide enriched composition obtained in step d), are subjected in step e) to an electrolysis.

Moreover, it is preferred that at least a portion and more preferably all the bromine containing composition being obtained in the electrolysis is recycled back into step a).

In a further development of the idea of the present invention, it is suggested that the electrolysis is performed in step e) by using an electrolytic cell comprising an anode, a cathode and a membrane sandwiched between the anode and the cathode, wherein an electric field can be applied between the anode and the cathode. The hydrogen bromide enriched composition obtained in step b) and/or (and preferably and) the hydrogen bromide enriched composition obtained in step d) is/are fed to the anode, optionally a second composition comprising hydrogen bromide and water is fed to the cathode, and the electrolytic cell is operated to produce hydrogen at the cathode, wherein the bromine containing composition is produced at the anode.

As set out in further detail below, the electrolysis is performed in step e) preferably by using an electrolytic cell comprising a membrane made of a fluoropolymer membrane having a glass transition temperature of at least 110°C.

Furthermore, it is preferred that the electrolysis is performed in step e) by operating the electrolysis cell at an operational temperature of at least 70°C, wherein the operational temperature is below the boiling point of the optional second composition fed to the cathode, and preferably at an operational temperature of 70°C to 122°C.

In addition, it is preferred that the electrolysis is performed in step e) by operating the electrolytic cell at an operational pressure, which increases from the anode to the cathode.

The anode and the cathode regularly comprise electroconductive material, especially metal, coated metal, carbon cloth, graphite felt, carbon fibre composite, carbon loaded polymer or graphite, and are preferably made of carbon fibre composite or coated metal. The membrane may be a sheet or layer which can be used in standard electrolysis cells preferably as a proton exchange membrane. Accordingly, preferably protons can migrate through such a membrane.

As set out above, the electrolysis is preferably performed with a liquid (i.e. aqueous) hydrogen bromide enriched composition being obtained in an absorption step performed in step b) and/or step d). However, alternatively, but less preferred a gaseous hydrogen bromide enriched composition may be used for the electrolysis in step e), if the absorbing agent is separated and removed from the hydrogen bromide before the electrolysis or if the separation of the hydrogen bromide in step b) and/or step d) is achieved by a different separation technique than absorption. In an aqueous hydrogen bromide containing composition, the hydrogen bromide is dissolved in water, wherein the hydrogen bromide dissolved in water will regularly be dissociated in the water so that the composition will comprises ionic species, such as especially protons, hydronium ions and bromine cations. At the anode, to which the hydrogen bromide is fed, protons are generated during the electrolysis, which then migrate through the membrane to the cathode. According to the present invention, an aqueous hydrogen bromide enriched composition may also be fed to the cathode. This allows to operate the electrolytic cell at a lower operational voltage, which reduces the operational costs. Furthermore, this allows to operate the electrolytic cell at higher temperatures, which lowers the cooling and/or heating requirements of the electrolysis cell, which also reduces the operational costs. Additionally, this allows to operate the electrolytic cell at higher current densities, which reduces the capital expenditures of the electrolysis cell. Moreover, this may reduce the concentration gradient across the membrane and thus reduce or even mitigate undesirable transfer of hydrogen bromide and/or bromine and/or bromine cations from the anode to the cathode through the membrane. Additionally, the presence of hydrogen bromide on the cathode side may allow hydrogen bromide to cross through the membrane from the cathode to the anode side. This results in an increase of the efficiency of the system and allows avoiding any perturbation during electrolysis. Without wishing to be bound to theory, it is assumed that in this embodiment there is actually a hydrogen bromide crossing in reverse direction, i.e., from the cathode to anode. Such a hydrogen bromide crossing in reverse direction is considered to be one of the reasons why the efficiency of the electrolysis of this embodiment can be increased. It is preferred in this embodiment that the hydrogen bromide concentration of the composition fed to the cathode is at least 0.5 mol/kg, more preferably at least 1 mol/kg, still more preferably at least 2 mol/kg, even more preferably at least 3 mol/kg, further preferably at least 4 mol/kg, even further preferably at least 5 mol/kg and most preferably at least 6 mol/kg, with mol/kg referring to the mol of hydrogen bromide per kilogram of the respective composition.

Independently, from whether a hydrogen bromide composition is also led to the cathode of the electrolysis cell or not, it is preferred that the electrolytic cell is operated during the electrolysis of step e) at an operational voltage Uₒₚ between more than 0 mV to 1,900 mV and preferably between more than 0 mV to 1,700 mV. Operating the electrolytic cell at such an operational voltage improves the selectivity of the reaction towards hydrogen bromide electrolysis and thereby reduce the operational costs of the electrolysis.

In a further development of the idea of the present invention it is preferred that the electrolysis in step e) is performed by operating the electrolytic cell at an operational temperature of 70°C to 122°C. Operating the electrolytic cell at such an operational temperature can lower the cooling and/or heating requirements. Such higher temperatures increase the proton conductivity of a fluoropolymer-comprising membrane, which allows to operate the electrolysis cell at higher current densities and/or at lower voltages. Higher current densities help to keep the capital expenditures low. It is thus particularly preferred that the operational temperature is maintained at more than 70°C, more preferably at more than 90°C and most preferably at more than 100°C.

It is further preferred that the electrolytic cell is operated during the electrolysis in step e) at an operational pressure, which increases from the anode to the cathode. In other words, there is a pressure gradient which increases from the anode to the cathode, wherein the respective pressures at the anode and the cathode have the same reference point, i.e., are either both absolute pressures, or are both gauge pressures relative to the atmosphere surrounding the electrolytic cell. In such a case, the electrolysis regularly occurs in the gaseous phase, i.e. by feeding gaseous streams to the anode and the cathode. The pressure at the cathode is in such a case higher than the pressure at the anode, preferably higher by at least 0.01 MPa (i.e. the difference between the pressure at the cathode and the pressure at the anode is 0.01 or more MPa), more preferably higher by at least 0.02 MPa, still more preferably higher by at least 0.05 MPa and most preferably higher by at least 0.1 MPa. As an example, when the absolute pressure at the anode is 0.1 MPa, the absolute pressure at the cathode is 0.11 to 0.3 MPa, more preferably 0.12 to 0.25 MPa, still more preferably 0.15 to 0.2 MPa and most preferably 0.2 MPa. However, optional pressures at the cathode which are higher than the pressure at the anode by 1 MPa, by 2 MPa, by 3 MPa, by 4 MPa or even by 5 MPa are also contemplated. With such increasing pressures, or pressure gradients, reduced operational voltages of the electrolytic cell can be achieved, which can lead to reduced operational costs. Additionally, the pressure gradient may allow to obtain the hydrogen at the cathode side at a higher pressure so that no or less compression may be required subsequently for densifying the obtained hydrogen. Additionally, the operation at differential pressure improves the operational parameters.

According to a further preferred embodiment of the present invention, the membrane used for the electrolysis of step e) is a fluoropolymer membrane. Good results are in particular obtained, when the fluoropolymer membrane has a glass transition temperature of at least 110°C, preferably of at least 120°C and more preferably of at least 125°C. Preferably, the fluoropolymer membrane (in a dry state) consists of the fluoropolymer and more preferably consists of the fluoropolymer in a chemically stabilized form. As used herein, a fluoropolymer is a fluorocarbon-based polymer with multiple carbon-fluorine bonds. Such a fluoropolymer is regularly free of silicon atoms. Such a fluoropolymer may further be free of chlorine, bromine and/or iodine atoms. As used herein, the glass transition temperature of the fluoropolymer membrane is determined according to DIN EN ISO 11357-2:2020-08 (half-step-height method). Accordingly, as used herein, the glass transition temperature of the fluoropolymer membrane is determined by a differential scanning calorimetry (DSC) measurement, more specifically from a curve obtained by such a DSC measurement. The glass transition temperature of the fluoropolymer membrane is determined on a sample of the fluoropolymer membrane, more specifically on a sample of the fluoropolymer membrane in the fully hydrated state. According to the present invention, a fluoropolymer membrane in a fully hydrated state is a fluoropolymer membrane, which does not take up any further (deionized) water (deionized H₂O, as described in ASTM D5127-13(2018) standard) at a temperature of 80°C after 24 hours, which is hence a fully hydrated fluoropolymer membrane, or a fluoropolymer membrane saturated with water. Hence, the water uptake of the fluoropolymer membrane has reached its maximum. A fully hydrated fluoropolymer membrane is obtained by immersing the fluoropolymer membrane in deionized water which will result in a weight gain of the fluoropolymer membrane as measurable by a balance. When there is no weight gain over time anymore, the fluoropolymer membrane is in the fully hydrated state. A fluoropolymer membrane in the fully hydrated state is regularly obtained by immersing the fluoropolymer membrane in deionized water having a temperature of 80°C for a time period of 24 hours. As used herein, the glass transition temperature refers to the lowest glass transition temperature of the fluoropolymer. It is known that certain polymers or membranes made of polymers, respectively, e.g., Nafion^{®}-type membranes, may have several glass transition temperatures, in particular two glass transition temperatures. The first glass transition temperature is due to the mobility of the main chain in the polymer matrix, while the second glass transition temperature is due to side chain effects, especially effects associated with strong interactions of functional groups in such side chains like sulfonic acid groups. Once the first glass transition temperature has been reached, the membrane undergoes irreversible changes and can in particular not be used for an electrolysis process anymore. Accordingly, in the context of the present invention, if a material, especially a membrane or membrane sample, exhibits several glass transition temperatures, only the lowest glass transition temperature is relevant for the glass transition temperature definitions provided herein.

A fluoropolymer membrane having a glass transition temperature at least 110°C allows to carry out the electrolysis at higher temperatures and/or at higher pressures because of a more resilient fluoropolymer membrane. Without wishing to be bound to theory, it is assumed that the resilience to hydrogen bromide and, after electrolysis, of hydrogen and bromine at higher temperatures and also at higher pressures is imparted by the fluoropolymer membrane having a glass transition temperature of at least 110°C. Moreover, especially higher temperatures can decrease the resistance of the fluoropolymer membrane which can allow for higher proton conductivities. Higher proton conductivities can allow for higher current densities at lower operational voltages. Higher current densities helps to keep the capital expenditures low, while lower operational voltages lead to lower operational costs.

It is preferred that the fluoropolymer of the membrane comprises -(CF₂-CF₂)- repeat units. Such -(CF₂-CF₂)- repeat units increase the resilience of the fluoropolymer membrane. An increased resilience allows that the hydrogen bromide electrolysis may be performed at higher temperatures and at higher pressures, which allow the operation at higher current densities and/or at lower voltages, thereby making the electrolysis more economical. Moreover, it is preferred that the fluoropolymer of the membrane does not comprise structural entities of the formula -O-CF₂-CF(CF₃)-O-. Such structural entities are present in commercial membranes sold under the tradename Nation^{®}, but may lead to less rigidity of the membrane, which is thus more prone to degradation at higher temperatures and higher pressures.

It is preferred that the fluoropolymer membrane, more specifically the fluoropolymer comprised by and preferably constituting the membrane, comprises -CF₃ chain ends. Such -CF₃ chain ends may increase the resilience of the fluoropolymer membrane. An increased resilience can allow for HBr electrolysis at higher temperatures and at higher pressures, with may allow operation at higher current densities and/or at lower voltages, thereby making the method according to the present invention more economical.

In accordance with a particular preferred embodiment of the present invention the fluoropolymer of the membrane is a sulfonated fluoropolymer. As used herein, sulfonated means that the fluoropolymer of the fluoropolymer membrane bears -SO₃H groups. Such -SO₃H groups increase the proton conductivity of the fluoropolymer membrane, which allows to operate the electrolysis at higher current densities and/or at lower voltages, thereby making the electrolysis more economical. It is particularly preferred that the sulfonated fluoropolymer comprises -O-(CF₂)ₙ-SO₃H groups, wherein n is an integer selected from 1, 2, 3, 4 and 5, preferably 2. Such -O-(CF₂)ₙ-SO₃H groups help to achieve an increased proton conductivity of the fluoropolymer membrane, while simultaneously increasing the resilience of the fluoropolymer membrane. An advantageous balance between these effects is in particular obtained, when n is selected from 1 to 5 and especially when n is 2. Without wishing to be bound to theory, it is assumed that the comparably short chain length of the -O-(CF₂)ₙ-SO₃H groups increases the glass transition temperature of the fluoropolymer membrane making it more resilient at higher temperatures. As such, the sulfonated fluoropolymer membrane is particularly advantageous from the viewpoint of hydrogen bromide electrolysis at higher temperatures and/or at higher pressures, and hence at higher current densities and/or at lower voltages. As such, the sulfonated fluoropolymer membrane makes the electrolysis particularly economical.

It is preferred that the sulfonated fluoropolymer membrane comprises a hydrolysed copolymer of F₂C=CF₂ and CF₂=CF-O-(CF₂)₂-SO₂F. Such a hydrolysed copolymer can lead to an increased proton conductivity and/or an increased resilience of the fluoropolymer membrane. As such, the sulfonated fluoropolymer membrane is particularly advantageous from the viewpoint of hydrogen bromide electrolysis at higher temperatures and/or at higher pressures, and hence at higher current densities and/or at lower voltages. As such, the sulfonated fluoropolymer membrane makes the electrolysis particularly economical.

It is further preferable that the fluoropolymer membrane has an acid capacity of at least 0.9 meq/g, more preferably between 0.95 and 1.5 meq/g and most preferably between 1.0 and 1.2 meq/g. As used herein, meq/g refers to milli-equivalent per gram, wherein the equivalent is mol -H (i.e., mol of H-acidic cites), preferably mol -SO₃H (i.e., mol of SOsH-groups). The acid capacity represents the total of active sites or functional groups responsible for proton exchange of the fluoropolymer membrane. Such an acid capacity can increase the proton conductivity and can thereby lead to an increased hydrogen bromide conversion in the electrolysis. An increased hydrogen bromide conversion reduces the need for a separation and possible recirculation of unconverted hydrogen, which can help to maintain low capital expenditures.

It is further preferred that the fluoropolymer membrane (in dry state) has a thickness of 25 to 350 µm, more preferably of 40 to 150 µm, still more preferably of 45 to 130 µm and most preferably of 45 to 55 µm. Such a thickness achieves a good balance between sufficient resilience on the one hand and low capital expenditures on the other hand. At a lower thickness, there is a risk of undesired crossover of hydrogen/bromine through the fluoropolymer membrane and at a higher thickness, an undesirable decrease in proton conductivity may result.

In a further development of the idea of the present invention, it is proposed that the fluoropolymer membrane (in dry state) has a density of at least 1.70 g/cm³, more preferably of at least 1.80 g/cm³, still more preferably of 1.80 to 2.00 g/cm³ and most preferably of 1.90 to 1.95 g/cm³. The density of the fluoropolymer membrane may be determined on a sample thereof according to ASTM D792-20. It is further preferred that the fluoropolymer membrane (in dry state) has a tensile modulus of 100 to 500 MPa, more preferably of 150 to 400 MPa, still more preferably of 200 to 300 MPa and most preferably of 260 to 280 MPa. The tensile modulus of the fluoropolymer membrane may be determined on a sample thereof according to ASTM D638-14. Moreover, it is preferred that the fluoropolymer membrane (in dry state) has a tensile stress at break in machine direction (MD), according to ASTM D882-18, of 10 to 70 MPa, more preferably of 20 to 60 MPa, still more preferably of 30 to 50 MPa and most preferably of 35 to 45 MPa. Also, it is preferred that the fluoropolymer membrane (in dry state) has a tensile stress at break in cross direction (CD), according to ASTM D882-18, of 5 to 60 MPa, more preferably of 10 to 50 MPa, still more preferably of 20 to 40 MPa and most preferably of 25 to 35 MPa. In addition, it is preferred that the fluoropolymer membrane (in dry state) has an elongation at break in machine direction (MD), according to ASTM D882-18, of 100 to 200%, more preferably of 120 to 180%, still more preferably of 140 to 160% and most preferably of 145 to 155%. Likewise, it is preferred that the fluoropolymer membrane (in dry state) has an elongation at break in cross direction (CD), according to ASTM D882-18, of 100 to 350%, more preferably of 120 to 300%, still more preferably of 150 to 250% and most preferably of 190 to 210%. The aforementioned properties increase the resilience of the fluoropolymer membrane. An increased resilience allows that the hydrogen bromide electrolysis may be performed at higher temperatures and at higher pressures, which may allow operation at higher current densities and/or at lower voltages, thereby making the electrolysis of step e) more economical.

The bromine containing composition being obtained during the electrolysis of step e) water and may contain more or less non-converted hydrogen bromide. Therefore, it is suggested in a further development of the idea of the present invention to purify the bromine containing composition being obtained during the electrolysis of step e), before preferably at least a portion thereof is recycled back into step a). Preferably, the purification comprises a distillation step. During the distillation, as bottom composition a mixture of hydrogen bromide and water is obtained and as overheads composition purified bromine is obtained. While the purified bromine may be further purified, for instance by subjecting it to a drying step to further reduce its water content, before preferably at least a portion and more preferably all of the so purified bromine is recycled back into step a), the mixture of water and hydrogen bromide being obtained as bottom composition of the distillation may be used as absorbing agent in the absorption steps. Optionally, residual traces of bromine may be removed from the bottom composition, before using it as absorbing agent, wherein the removal of the residual traces of bromine may be performed by treating the bottom composition with an oxidizable compound, such as a compound being selected from the group consisting of hydrogen, carbon monoxide containing compositions, ammonia, hydrogen sulfide, sulfur dioxide and arbitrary combinations of two or more of the aforementioned compounds. For instance, the mixture of hydrogen bromide and water being obtained as bottom composition of the distillation and being optionally dried afterwards contains 50 to 99% by weight of water and 1 to 50% by weight of hydrogen bromide.

The hydrogen being produced during the electrolysis of step e) may be completely withdrawn as product from the process or a portion of the produced hydrogen may be recycled into the dehydrobromination reaction performed in step c).

As set out above, step b) as well as step d) may comprise the generation of a polybromopropane enriched composition. Preferably, the polybromopropane enriched composition is subjected to a thermal oxidization in a thermal oxidizer in order to burn or oxidize, respectively, the polybromopropanes contained in the polybromopropane enriched composition so as to recover bromine. The so recovered bromine is preferably - together with the hydrogen enriched compositions - led and subjected to the electrolysis of step e). More specifically, the enriched polybromopropane composition or a part thereof is contacted with oxygen at high temperature of more than 700°C, during which the polybromopropanes are transformed into a gaseous mixture comprising carbon dioxide, hydrogen bromide and bromine. The resulted gas stream is then preferably subjected to an absorption step with aqueous hydrogen bromide so as to separate the hydrogen bromide and the bromine from the rest of the gaseous products. The obtained liquid stream containing the recovered hydrogen bromide and bromine is then preferably sent to the electrolysis.

Alternatively, the polybromopropane enriched composition may be reacted, thermally or in the presence of a catalyst, to bromopropane. For instance, the catalyst may comprise a metal being selected from the group of transition metals, such as molybdenum, cobalt, iron, nickel, copper or silver, and of noble metals, such as platinum, ruthenium or palladium, wherein the metal is supported on a carrier, such as a carrier made of aluminium oxide, silicon oxide, titanium oxide, cerium oxide or carbon. Particularly preferably, the metal is platinum, palladium or a mixture of platinum and palladium. In a further development of this embodiment, it is preferred that the reaction of the polybromopropane enriched composition to bromopropane is performed in the presence of hydrogen. Good results are in particular obtained, when the molar ratio of polybromopropane to hydrogen is 10:1 to 1:10, more preferably 5:1 to 1:5, still more preferably 2:1 to 1:2 and most preferably about 1:1.

In accordance with still an alternative embodiment, the polybromopropane enriched composition is completely hydrogenated in the presence of hydrogen and of a suitable catalyst to propane and/or higher alkanes.

In accordance with still an alternative embodiment, the enriched polybromopropane composition or a part thereof is subjected in to a hydrotreatment step on a platinum-containing catalyst at a temperature at least 300°C, at a pressure of at least 2 barg with a molar ration of hydrogen/feed of at least 1 so as to produce a mixture of propane, propylene, bromopropane products from the enriched polybromopropane compositions. The resulted mixture can be further sent to the separation step b), such as to a second column used in the separation step b) as described as preferred further above.

In accordance with another aspect the present invention relates to a plant for producing propylene and hydrogen from propane, which comprises:
a) a (bromination) reactor for reacting a mixture comprising propane and bromine so as to produce a first reaction mixture containing bromopropane and hydrogen bromide, wherein the reactor comprises an inlet line for the mixture and an outlet line for the first reaction mixture,
b) a first separation unit comprising an inlet line for the first reaction mixture being connected with the outlet line for the first reaction mixture of the reactor a), an outlet line for a bromopropane enriched composition and an outlet line for a hydrogen bromide enriched composition,
c) a (dehydrobromination) reactor for catalytically reacting the bromopropane enriched composition so as to produce a second reaction mixture containing propylene and hydrogen bromide, wherein the reactor comprises an inlet line for the bromopropane enriched composition being connected with the outlet line for the for a bromopropane enriched composition of the separation unit b), and an outlet line for the second reaction mixture,
d) a second separation unit comprising an inlet line for the second reaction mixture being connected with the outlet line for the second reaction mixture of the reactor c), an outlet line for propylene and an outlet line for a hydrogen bromide enriched composition, and
e) an electrolysis cell comprising an anode, a cathode and a membrane sandwiched between the anode and the cathode as well as an inlet line for a hydrogen bromide containing composition, an outlet line for hydrogen and an outlet line for a bromine containing composition, wherein the inlet line of the electrolysis cell is connected with the outlet line for the hydrogen bromide enriched composition of the first separation unit and/or with the outlet line for the hydrogen bromide enriched composition of the second separation unit.

Preferably, the outlet line for the bromine containing composition of the electrolysis cell e) is connected with the inlet line of the reactor a).

The outlet line for the bromine containing composition of the electrolysis cell e) may be connected directly with the inlet line of the reactor a) or indirectly, i.e. with another device or unit, such as a purification unit, being arranged between the bromine containing composition of the electrolysis cell e) and the inlet line of the reactor a).

In a further development of the idea of the present invention, it is proposed that the first separation unit comprises at least one distillation column and/or at least one absorption column. More preferably, the first separation unit comprises at least one distillation column and at least one absorption column and still more preferably the first separation unit comprises two distillation columns and one absorption column.

In accordance with a particular preferred embodiment of the present invention, the first separation unit comprises:
- a first distillation column comprising an inlet line being connected with the outlet line for the first reaction mixture of the reactor a), an overheads outlet line and a bottom outlet line,
- an absorption column comprising an inlet line being connected with the overheads outlet line of the first distillation column, an inlet line for absorbing agent, an outlet line for a hydrogen bromide enriched composition and an outlet line for a purified propane composition and
- a second distillation column comprising an inlet line being connected with the bottom outlet line of the first distillation column, an overheads outlet line for the bromopropane enriched composition and a bottom outlet line.

In accordance with a further particular preferred embodiment of the present invention, the reactor c) further comprises an inlet line for hydrogen. Alternatively, the plant comprises a line for hydrogen leading into the inlet line for the bromopropane enriched composition of the reactor a).

Furthermore, it is preferred that the outlet line for a purified propane composition is directly or indirectly connected with the inlet line of the bromination reactor a).

Likewise to the first separation unit, it is preferred that the second separation unit comprises at least one distillation column and/or at least one absorption column. More preferably, the second separation unit comprises at least one distillation column and at least one absorption column and still more preferably the second separation unit comprises two distillation columns and one absorption column.

Good results are in particular obtained, when the second separation unit comprises:
- a first distillation column comprising an inlet line being connected with the outlet line for the second reaction mixture of the reactor c), an overheads outlet line and a bottom outlet line,
- an absorption column comprising an inlet line being connected with the overheads outlet line of the second distillation column, an inlet line for absorbing agent, an outlet line for a hydrogen bromide enriched composition and an outlet line for propylene or for a composition comprising propylene and hydrogen and
- a second distillation column comprising an inlet line being connected with the bottom outlet line of the first distillation column, an overheads outlet line and a bottom outlet line.

Furthermore, it is preferred that the second separation unit additionally comprises a separation device comprising an inlet line being connected with the outlet line for the composition comprising propylene and hydrogen of the absorption column, an outlet line for hydrogen and an outlet line for propylene, wherein the separation device is preferably a pressure swing adsorption column.

In a further development of the idea of the present invention, it is suggested that the plant further comprises an oxidizing unit and an absorption column. While the oxidizing unit comprises an inlet line for air, an inlet line being connected with the bottom outlet line of the second distillation column of the first separation unit and being connected with the bottom outlet line of the second distillation column of the second separation unit, and an outlet line, the absorption column comprises an inlet line being connected with the outlet line of the oxidizing unit, an inlet line being connected with the outlet line for the hydrogen bromide enriched composition of the absorption column of the first separation unit and being connected with the outlet line for the hydrogen bromide enriched composition of the absorption column of the second separation unit, an outlet line for air and an outlet line for hydrogen bromide enriched composition being connected with the inlet line for hydrogen bromide containing composition of the electrolysis cell e).

Preferably, the plant further comprises a purification unit for obtaining purified bromine from the bromine containing composition obtained in the electrolysis cell e). Good results are in particular obtained, when the purification unit comprises a distillation column comprising an inlet line being connected with the outlet line for the bromine containing composition of the electrolysis cell e), an overheads outlet line and a bottom outlet line. It is further preferred that the purification unit further comprises a dryer comprising an inlet line being connected with the overheads outlet line of the distillation column and an outlet line for purified bromine, which is connected with the inlet line of the reactor a).

Specific embodiments in accordance with the present invention are subsequently described with reference to the appended drawings and by examples.
- Fig. 1: is a schematic view of a plant for producing propylene and hydrogen from propane in accordance with one embodiment of the present invention.
- Fig. 2: shows the results of example 3.
- Fig. 3: shows the results of example 4.

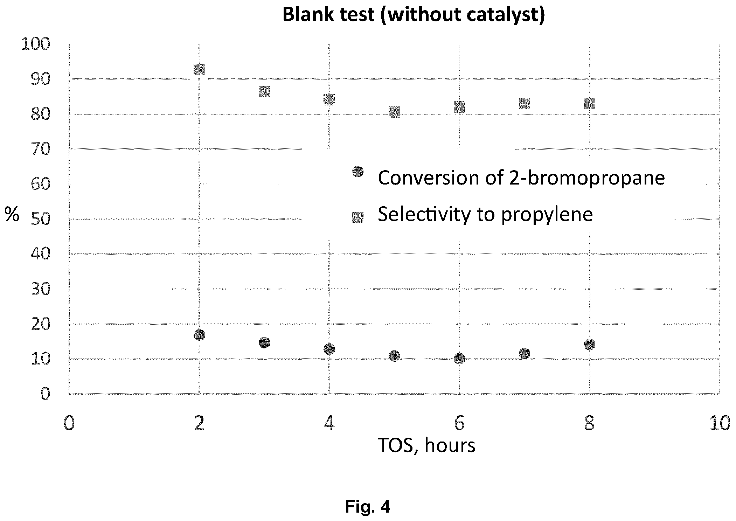

- Fig. 4: shows the results of comparative example 1.

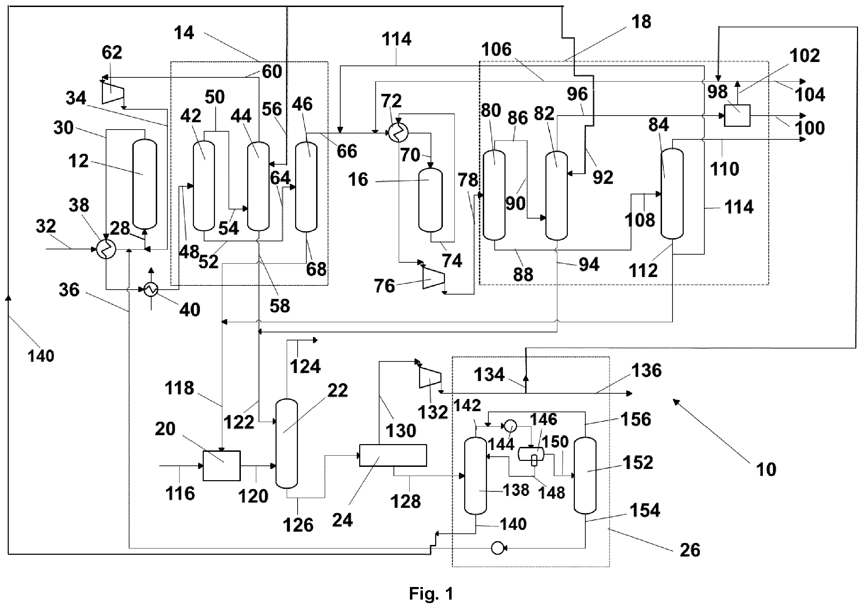

The plant 10 shown in figure 1 comprises:
i) a bromination reactor 12 for reacting a mixture comprising propane and bromine so as to produce a first reaction mixture containing bromopropane and hydrogen bromide,
ii) a first separation unit 14 for separating from the first reaction mixture a bromopropane enriched composition, a propane enriched composition, a polybromopropane enriched composition and a hydrogen bromide enriched composition,
iii) a dehydrobromination reactor 16 for catalytically reacting the bromopropane enriched composition being obtained in the first separation unit 14 so as to obtain a second reaction mixture containing propylene and hydrogen bromide,
iv) a second separation unit 18 for separating from the second reaction mixture propylene, a polybromopropane enriched composition and a hydrogen bromide enriched composition,
v) a thermal oxidizer 20 for oxidizing the polybromopropane enriched compositions being obtained in the first and second separation units,
vi) an absorption column 22 for contacting the hydrogen bromide enriched compositions being obtained in the first and second separation units and the oxidization product obtained in the thermal oxidizer 20,
vii) an electrolysis cell 24 for producing from the hydrogen bromide enriched composition being treated in the absorption column 22 hydrogen and a bromine containing composition and
viii) a purification unit 26 for obtaining pure bromine from the bromine containing composition being obtained in the electrolysis cell 24.

More specifically, the bromination reactor 12 comprises an inlet line 28 for the mixture comprising propane and bromine and an outlet line 30 for the first reaction mixture. The inlet line 28 is connected with a feed line 32 for a propane containing gas, such as natural gas, a recycle line 34 for propane and a recycle line 36 for bromine, wherein a heat exchanger 38 is arranged so that the first reaction mixture being withdrawn from the bromination reactor 12 via the outlet line 30 preheats the propane containing gas being led through the feed line 32. The outlet line 30 passes a further heat exchanger 40, before it leads into the first separation unit 14. The first separation unit 14 comprises, seen form the upstream to the downstream direction of the first separation unit 14, a first distillation column 42, an absorption column 44 and a second distillation column 46. While the first distillation column 42 comprises an inlet line 48 being connected with the outlet line 30 for the first reaction mixture of the bromination reactor 12, an overheads outlet line 50 and a bottom outlet line 52, the absorption column 44 comprises an inlet line 54 being connected with the overheads outlet line 50 of the first distillation column 42, an inlet line 56 for absorbing agent, an outlet line 58 for a hydrogen bromide enriched composition and an outlet line for the purified propane composition 60. The outlet line for the purified propane composition 60 leads via a compressor 62 into the recycle line 34 for propane. In turn, the second distillation column 46 comprises an inlet line 64 being connected with the bottom outlet line 52 of the first distillation column 42, an overheads outlet line 66 for the bromopropane enriched composition and a bottom outlet line 68 for a polybromopropane enriched composition.

The dehydrobromination reactor 16 for catalytically reacting the bromopropane enriched composition so as to produce a second reaction mixture containing propylene and hydrogen bromide comprises an inlet line 70 for the bromopropane enriched composition being connected via a heat exchanger 72 with the overheads outlet line 66 for bromopropane enriched composition of the second distillation column 46 of the first separation unit 14, and an outlet line 74 for the second reaction mixture, which leads via the heat exchanger 72 and a compressor 76 into the second separation unit 18. The second separation unit 18 comprises, seen form the upstream to the downstream direction of the second separation unit 18, a first distillation column 80, an absorption column 82 and a second distillation column 84. While the first distillation column 80 comprises an inlet line 78 for the second reaction mixture of the bromination reactor 12 being connected with the outlet line 74 of the dehydrobromination reactor 16, an overheads outlet line 86 and a bottom outlet line 88, the absorption column 82 comprises an inlet line 90 being connected with the overheads outlet line 86 of the first distillation column 80, an inlet line 92 for absorbing agent, an outlet line 94 for a hydrogen bromide enriched composition and an outlet line for the (purified) propylene 96. The outlet line for the (purified) propylene 96 leads into a pressure swing adsorption column 98, which comprises an outlet line 100 for propylene as well as an outlet line 102 for hydrogen splitting into a withdrawal line 104 for hydrogen and into a recycle line 106 leading back into the inlet line 70 for the second reaction mixture of the dehydrobromination reactor 16. In turn, the second distillation column 84 of the second separation unit 18 comprises an inlet line 108 being connected with the bottom outlet line 88 of the first distillation column 80, an overheads outlet line 110 for a C₄₋₅-hydrocarbon composition, a bottom outlet line 112 for a polybromopropane enriched composition and a recycle line 114 for a polybromopropane enriched composition leading into the overheads outlet line 66 of the second distillation column 44 of the first separation unit 14.

The oxidizing unit 20 comprises an inlet line 116 for air, an inlet line 118 being connected with the bottom outlet line 68 of the second distillation column 46 of the first separation unit 14 and being connected with the bottom outlet line 112 of the second distillation column 84 of the second separation unit 18, and an outlet line 120. In turn, the absorption column 22 comprises an inlet line being connected with the outlet line 120 of the oxidizing unit 20, an inlet line 122 for hydrogen bromide enriched composition being connected with the outlet line 58 for the hydrogen bromide enriched composition of the absorption column 44 of the first separation unit 14 and being connected with the outlet line 94 for the hydrogen bromide enriched composition of the absorption column 82 of the second separation unit 18, an outlet line 124 for air and an outlet line 126 for hydrogen bromide enriched composition being connected with the inlet line for hydrogen bromide containing composition of the electrolysis cell 24. The electrolysis cell 24 comprises an outlet line 128 for a bromine containing composition as well as an outlet line 130 for hydrogen, which is connected with a compressor 132, a recycle line 134 leading into the recycle line 106 and a hydrogen removal line 136. Finally, the purification unit 26 comprises a distillation column 138, which comprises an inlet line being connected with the outlet line 128 for the bromine containing composition of the electrolysis cell 24, a bottom outlet line and recycle line 140 for a mixture of water and hydrogen bromide and an overheads outlet line 142 for hydrogen. While the bottom outlet line and recycle line 140 of the purification unit 26 leads into the inlet lines 56, 92 for absorbing agent of the second distillation columns 44, 82 of the first and second separations units 14, 18, the overheads outlet line 142 leads into a condenser 144 and from there into a vessel 146, from which a return line 148 leads back into the distillation column 138 and an outlet line 150 leads into a dryer column 152. The dryer column 152 comprises a bottom outlet line for bromine, which is connected with the recycle line 36, and an overheads outlet 156, which leads back into the overheads outlet line 142 of the distillation column 138.

During the operation of the plant 10, a mixture comprising propane and bromine is led via line 28 into the bromination reactor 12, in which the propane and bromine react to bromopropane and hydrogen bromide. In addition, the (first) reaction mixture comprises non-reacted propane, non-reacted bromine and side-products, such as polybromopropanes. Then, the (first) reaction mixture is led via lines 30, 48 into the first distillation column 42 of the first separation unit, in which the reaction mixture is separated into a bottom composition mainly containing bromopropanes and minor amounts of polybromopropanes as well as into an overheads composition, which mainly contains hydrogen bromide, non-reacted propane and non-reacted bromine. The overheads composition is fed via lines 50, 54 into the absorption column 44 and is contacted there with absorbent agent, which is an aqueous solution containing minor amounts of hydrogen bromide recycled via lines 140, 56 into the absorption column 44, so as to obtain a purified propane composition, which is recycled into the mixture used in step a) via lines 60, 34 and the compressor 62, and a hydrogen bromide enriched composition being withdrawn from the absorption column 44 via line 58. The bottom composition of the first distillation column 42 of the first separation unit 14 mainly containing bromopropanes and minor amounts of polybromopropanes is led via 52, 64 into the second distillation column 46, in which the polybromopropanes are separated as bottom composition from the bromopropanes. While the polybromopropane enriched composition is withdrawn from the second distillation column 46 via line 68, the bromopropane enriched composition is withdrawn from the second distillation column 46 via line 66. Then, the bromopropane enriched composition is dehydro-brominated in the dehydrobromination reactor 16 in the presence of hydrogen so as to obtain a second reaction mixture containing propylene, hydrogen bromide, hydrogen and polybromopropanes. The second reaction mixture is then separated in the second separation unit 18 into pure propylene, which is withdrawn via line 100, into pure hydrogen, which is withdrawn via line 102 and partially recycled into the dehydrobromination reactor 16 via lines 102, 106, 70, into a polybromopropane enriched composition, which is withdrawn via line 112, and into a hydrogen bromide enriched composition, which is withdrawn via line 94. The two polybromopropane enriched compositions being withdrawn from the first and second separation units via lines 68, 112 are oxidized in the thermal oxidizer 20 mainly to bromine, which is then together with the two hydrogen bromide enriched compositions being withdrawn from the first and second separation units via lines 58, 94 contacted in the absorption column 22, from which via line 126 hydrogen bromide enriched composition (which is in fact an aqueous solution containing hydrogen bromide) is withdrawn and led into the electrolysis cell 24. The electrolysis cell 24 produces from this composition at the cathode hydrogen and at the anode bromine. While the hydrogen is withdrawn from the electrolysis cell 24 via line 130 and partially recycled into the dehydrobromination reactor 16 via lines 134, 106, 70, the bromine containing composition, which is an aqueous solution containing bromine and hydrogen bromide, is withdrawn from the electrolysis cell 24 via line 128. Afterwards, the bromine containing composition is separated in the distillation column 138 into an aqueous solution containing hydrogen bromide, which is withdrawn via line 140 and recycled via lines 56, 92 as absorbing agent into the absorption columns 44, 82 of the first and second separation units 14, 18, and into a bromine enriched composition. The latter is withdrawn from the distillation column 138 via line 142 and dewatered in the distillation column 152 so as to obtain pure bromine, which is led via lines 154, 36 into the mixture being introduced via line 28 into the bromination reactor 12.

### Examples

### Example 1

### (Bromination tests - impact of pressure on bromine conversion)

A mixture of propane and bromine in a molar ratio 2.4:1 was reacted at a reaction temperature of 250°C at different pressures as summarized in the below table 1 and the residence time was measured, which was necessary to reach a full conversion of bromine. The results are also shown in the below table 1.

**Table 1**

| **Run** | **Pressure (bar)** | **Residence time required to reach full bromine conversion (seconds)** |
|---|---|---|
| 1 | 1 | 120 |
| 2 | 2 | 90 |
| 3 | 4 | 57 |
| 4 | 6 | 46 |
| 5 | 8 | 40 |
| 6 | 10 | 36 |
| 7 | 13 | 32 |
| 8 | 15 | 32 |

The results show that the pressure during the bromination reaction has an impact on the bromine conversion, on the selectivity of the reaction to polybrominated compounds and to unsaturated compounds, such as propylene and allyl bromide. Unsaturated compounds could be produced thermally at low partial pressure of below 4 bars due to a longer residence time required to fully convert the bromine. The produced (unsaturated) propylene significantly complicates the recycling of propane and should be avoided. Typically, a higher residence time leads to a higher concentration of polybrominated and unsaturated compounds.

### Example 2

### (Bromination tests - impact of ratio of propane to bromine)

Different mixtures of propane and bromine with the compositions and stream velocities summarized in the below table 2 were reacted in an adiabatic pipe reactor with inlet and outlet temperatures as summarized in the below table 2. The pressure within the reactor was 13 bar.

**Table 2**

| **Run** | **Ratio Propane to Br₂** | **Inlet Temp. (°C)** | **Outlet Temp. (°C)** | **Propane inlet (kmol/h)** | **CsBr at outlet (kmol/h)** | **C3 Conversion** | **Selectivity to C₃Br** | **Selectivity to C3Br2** |
|---|---|---|---|---|---|---|---|---|
| 1 | 0.5 | 250 | 661 | 200 | 0 | 100.00% | 3.72% | 96.28% |
| 2 | 1 | 250 | 524 | 200 | 27.54 | 86.23% | 84.03% | 15.97% |
| 3 | 2 | 250 | 413 | 200 | 105.20 | 47.40% | 94.84% | 5.16% |
| 4 | 2.5 | 250 | 387 | 200 | 123.05 | 38.48% | 96.04% | 3.96% |
| 5 | 3 | 250 | 367 | 200 | 135.47 | 32.26% | 96.67% | 3.33% |
| 6 | 4 | 250 | 342 | 200 | 151.16 | 24.42% | 97.63% | 2.37% |

The results show that the higher molar ratio of propane to bromine, the lower the propane conversion, but the higher the selectivity to bromopropane.

### Example 3

### (Catalytic test: Dehydrobromination of bromopropane)

Two grams of the gamma-aluminium oxide having a specific surface area of 180 m²/g and having a content of sodium compounds of less than 100 ppm and having a silicon dioxide content of less than 100 ppm were incipiency wetness impregnated with a solution of iron(III)nitrates to obtain about 5% by weight of iron on the catalyst. The material was dried for 24 h at 100°C and subjected to calcination at 550°C for 5 h in an air flow (ramp = 5 °C and hold = 5 h). The obtained material was pelletized and then crushed and seized to a 35 to 45 mesh fraction. Two grams of the obtained fraction of the catalyst containing 5wt% of Fe on alumina were loaded in a fixed-bed tubular quartz reactor with an inner diameter of 10 mm. The dead volume above the catalyst in the reactor was filled with the spherical quartz beads. The loaded catalyst was heated up in 3 Nl/h N₂ flow to 450°C at atmospheric pressure followed by stopping the nitrogen flow and switching to 3 Nl/h of hydrogen for 1 h. Then, the reactor was cooled down from 450°C in hydrogen flow to the reaction temperature, the H₂ flow was increase to 48.6 ml/min and the gaseous bromopropane (16 g/h) was sent in the reactor.

The conversion and selectivity to propylene (i.e. the catalyst performance) in the catalytic dehydrobromination of 2-bromopropane on the prepared above catalyst were measure for 50 hours -on-stream at a temperature 300°C, WHSV (2-bromopropane)- 8 h⁻¹ at a molar ratio of H₂-2-bromopropane of 1. Then, the temperature was increased to 350°C and the performance was measured for 25 additional hours-on-stream. Afterwards, the hydrogen flow was replaced by the same flow on N₂ and the test was continued for 25 additional hours -on-stream keeping the rest of the parameters the same.

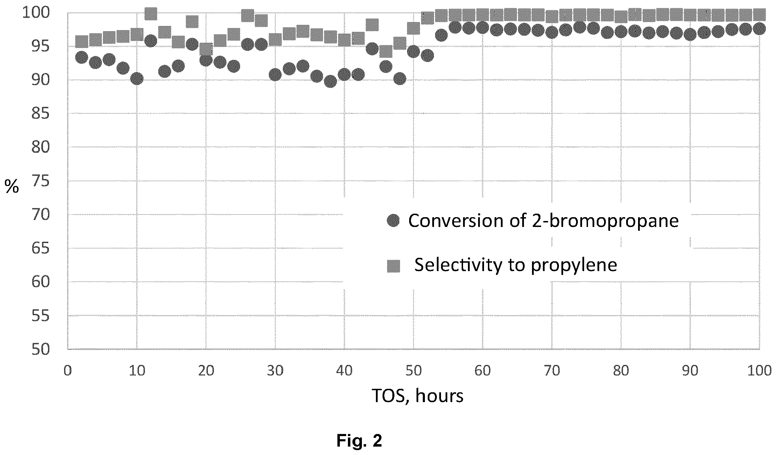

The results are shown in figure 2. The abscissa shows the time on stream in hours and the ordinate shows the % of the conversion (circle data points) and of the selectivity (square data points).

The results show the stable conversion close to complete of bromopropane and a very high selectivity to propylene of above 99%.

### Example 4

### (Catalytic test: Dehydrobromination of bromopropane)

Two grams of the gamma-aluminium oxide having a specific surface area of 180 m²/g, having a content of sodium compounds of less than 100 ppm and having a silica content of less than 100 ppm were incipiency wetness impregnated with a solution of cobalt nitrates so as to obtain about 5% by weight of cobalt on the catalyst. The material was dried for 24 h at 100°C and subjected to calcination at 550°C for 5 h in an air flow (ramp = 5 °C and hold = 5 h). The obtained material was pelletized and then crushed and seized to a 35 to 45 mesh fraction. Two grams of the obtained fraction of the catalyst containing 5% bby weight of Co on alumina were loaded in a fixed-bed tubular quartz reactor with an inner diameter of 10 mm. The dead volume above the catalyst in the reactor was filled with the spherical silica beads. The loaded catalyst was heated up in 3 Nl/h N₂ flow to 450°C at atmospheric pressure followed by stopping the nitrogen flow and switching to 3 Nl/h of hydrogen for 1 h. Then, the reactor was cooled down from 450°C in hydrogen flow to the reaction temperature, the hydrogen flow was increase to 48.6 ml/min and the gaseous bromopropane (16 g/h) was sent in the reactor.

The conversion and selectivity to propylene (the catalyst performance) in the catalytic dehydrobromination of 2-bromopropane on the prepared above catalyst were measured for about 200 hours on-stream at temperature 350°C, WHSV (2-bromopropane) 8 h⁻¹, H₂-2-bromopropane molar ratio of 1.

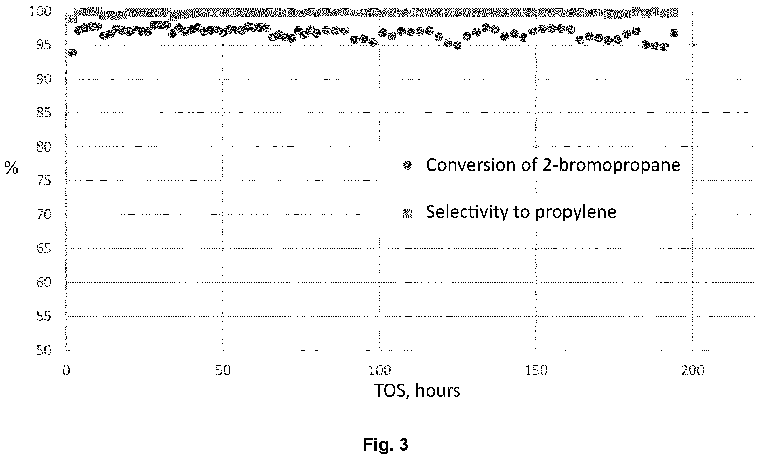

The results are shown in figure 3. The abscissa shows the time on stream in hours and the ordinate shows the % of the conversion (circle data points) and of the selectivity (square data points).

The results show the stable conversion close to complete of bromopropane and a very high selectivity to propylene, above 99%.

### Comparative Example 1

### (Blank test without a catalyst: Dehydrobromination of bromopropane)

The same reactor as in example 4 was filled with the spherical silica beads (the same inert materials, as used in the catalytic tests). The loaded with silica beads reactor was heated up in 3 Nl/h N₂ flow to 450°C at atmospheric pressure followed by stopping the nitrogen flow and switching to 3 Nl/h of hydrogen for 1 h. Then, the reactor was cooled down from 450°C in hydrogen flow to the reaction temperature, the hydrogen flow was increase to 48.6 ml/min and the gaseous bromopropane (16 g/h) was sent in the reactor.

The conversion and selectivity to propylene (the catalyst performance) in the catalytic dehydrobromination of 2-bromopropane on the prepared above catalyst were measure at temperature 350°C, H₂-2-bromopropane molar ratio of 1.

The results are shown in figure 5. The abscissa shows the time on stream in hours and the ordinate shows the % of the conversion (circle data points) and of the selectivity (square data points).

The results clearly show a very low conversion and much lower selectivity. So, the thermal gas phase reaction is insignificant. This shows that the process requires a catalyst.

### Reference Numeral List

- 10: Plant
- 12: Bromination reactor
- 14: First separation unit
- 16: Dehydrobromination reactor
- 18: Second separation unit
- 20: Thermal oxidizer
- 22: Absorption column
- 24: Electrolysis cell
- 26: Purification unit
- 28: Inlet line for the first reaction mixture of the bromination reactor
- 30: Outlet line of the reactor
- 32: Feed line for a propane containing gas
- 34: Recycle line for propane
- 36: Recycle line for bromine
- 38: Heat exchanger
- 40: Heat exchanger
- 42: First distillation column of the first separation unit
- 44: Absorption column of the first separation unit
- 46: Second distillation column of the first separation unit
- 48: Inlet line of the first distillation column of the first separation unit
- 50: Overheads outlet line of the first distillation column
- 52: Bottom outlet line of the first distillation column
- 54: Inlet line of the absorption column
- 56: Inlet line for absorbing agent
- 58: Outlet line for a hydrogen bromide enriched composition
- 60: Outlet line for a purified propane composition
- 62: Compressor
- 64: Inlet line of the second distillation column
- 66: Overheads outlet line of the second distillation column
- 68: Bottom outlet line of the second distillation column
- 70: Inlet line for the second reaction mixture of the dehydrobromination reactor
- 72: Heat exchanger
- 74: Outlet line for second reaction mixture
- 76: Compressor
- 78: Inlet line of the first distillation column of the second separation unit
- 80: First distillation column of the second separation unit
- 82: Absorption column of the second separation unit
- 84: Second distillation column of the second separation unit
- 86: Overheads outlet line of the first distillation column
- 88: Bottom outlet line of the first distillation column
- 90: Inlet line of the absorption column
- 92: Inlet line for absorbing agent
- 94: Outlet line for a hydrogen bromide enriched composition
- 96: Outlet line for purified propylene
- 98: Pressure swing adsorption column
- 100: Outlet line for propylene
- 102: Outlet line for hydrogen
- 104: Withdrawal line for hydrogen
- 106: Recycle line for hydrogen
- 108: Inlet line of the second distillation column of the second separation unit
- 110: Overheads outlet line for a C₄₋₅-hydrocarbon composition
- 112: Bottom outlet line of the second distillation column of the second separation unit
- 114: Recycle line for polybromopropane enriched composition

- 116: Inlet line for air
- 118: Inlet line for polybromopropane enriched composition
- 120: Outlet line of thermal oxidizer
- 122: Inlet line for hydrogen bromide enriched composition
- 124: Outlet line for air
- 126: Outlet line for hydrogen bromide enriched composition
- 128: Outlet line for bromine containing composition
- 130: Outlet line for hydrogen
- 132: Compressor
- 134: Recycle line for hydrogen
- 136: Hydrogen removal line
- 138: Distillation column of purification unit
- 140: Bottom outlet line for a mixture of water and hydrogen bromide
- 142: Overheads outlet line for hydrogen
- 144: Condenser
- 146: Vessel
- 148: Return line
- 150: Outlet line of vessel
- 152: Dryer column
- 154: Bottom outlet of dryer column
- 156: Overheads outlet of dryer column

## Claims

1. A process for producing propylene and hydrogen from propane comprising the following steps:
a) reacting a mixture comprising propane and bromine so as to produce a first reaction mixture containing bromopropane and hydrogen bromide,
b) subjecting the first reaction mixture obtained in step a) to at least one separation step so as to obtain a bromopropane enriched composition and to obtain a hydrogen bromide enriched composition,
c) catalytically reacting the bromopropane enriched composition obtained in step b) so as to produce a second reaction mixture containing propylene and hydrogen bromide,
d) subjecting the second reaction mixture obtained in step c) to at least one separation step so as to obtain a hydrogen bromide enriched composition and to obtain propylene and
e) subjecting the hydrogen bromide enriched composition obtained in step b) and/or the hydrogen bromide enriched composition obtained in step d) to an electrolysis so as to obtain hydrogen and a bromine containing composition.

2. The process in accordance with claim 1, wherein the mixture comprising propane and bromine used in step a) contains 10 to 90% by volume and preferably 50 to 80% by volume of propane and 10 to 50% by volume and preferably 20 to 45% by volume of bromine, wherein the molar ratio of propane to bromine in the mixture reacted used in step a) is 10:1 to 1:1 and preferably 3:1 to 2:1.

3. The process in accordance with claim 1 or 2, wherein the mixture is reacted in step a) at a temperature of 200 to less than 420°C, preferably of 230 to 410°C, more preferably of 240 to 400°C and most preferably of 240 to 300°C, wherein the mixture is reacted in step a) at a pressure of at least 400 kPa, preferably of at least 1 MPa and most preferably of 1 to 4 MPa, and wherein the residence time of the mixture in step a) is at least 10 seconds and preferably 20 seconds to 2 minutes.

4. The process in accordance with any of the preceding claims, wherein step b) comprises at least one and preferably two distillation steps for separating bromopropane from the first reaction mixture so as to obtain the bromopropane enriched composition, and wherein step b) comprises at least one absorption step for separating hydrogen bromide from the first reaction mixture so as to obtain the hydrogen bromide enriched composition.

5. The process in accordance with any of the preceding claims, wherein the bromopropane enriched composition obtained in step b) is reacted in step c) at a temperature of 250 to 450°C and in the presence of a catalyst, which is selected from the group consisting of alumina, silica, silicon oxide-aluminium oxides, zeolites with a Si/AI ratio above 25, titanium oxides, zirconium oxides and arbitrary combinations of two or more of the aforementioned materials.

6. The process in accordance with any of the preceding claims, wherein the bromopropane enriched composition obtained in step b) is reacted in step c) in the presence of hydrogen, wherein the molar ratio of bromopropane and hydrogen is preferably 10:1 to 1:10 and more preferably 5:1 to 1:5.

7. The process in accordance with any of the preceding claims, wherein step d) comprises at least one and preferably two purification steps and preferably distillation steps for separating propylene from the second reaction mixture so as to obtain the propylene, and wherein step d) comprises at least one absorption step for separating hydrogen bromide from the second reaction mixture so as to obtain the hydrogen bromide enriched composition.

8. The process in accordance with any of the preceding claims, wherein in step e) the hydrogen bromide enriched composition obtained in step b) and the hydrogen bromide enriched composition obtained in step d) are subjected to an electrolysis.

9. The process in accordance with any of the preceding claims, wherein the electrolysis is performed in step e) by using an electrolytic cell comprising an anode, a cathode and a membrane sandwiched between the anode and the cathode, wherein the hydrogen bromide enriched composition is fed to the anode, optionally a second composition comprising hydrogen bromide and water is fed to the cathode, and the electrolytic cell is operated to produce hydrogen at the cathode, wherein the bromine containing composition is produced at the anode.

10. The process in accordance with any of the preceding claims, wherein the electrolysis is performed in step e) by using an electrolytic cell comprising a membrane made of a fluoropolymer membrane having a glass transition temperature of at least 110°C, wherein the electrolysis is performed in step e) by operating the electrolysis cell at an operational temperature of at least 70°C, wherein the operational temperature is below the boiling point of the optional second composition fed to the cathode, and preferably at an operational temperature of 70°C to 122°C, and wherein preferably the electrolysis is performed in step e) by operating the electrolytic cell at an operational pressure, which increases from the anode to the cathode.

11. The method according to at least one of the preceding claims, wherein at least a portion and preferably all of the bromine containing composition being obtained during the electrolysis of step e) is recycled back into step a).

12. A plant for producing propylene and hydrogen from propane comprising:
a) a reactor for reacting a mixture comprising propane and bromine so as to produce a first reaction mixture containing bromopropane and hydrogen bromide, wherein the reactor comprises an inlet line for the mixture and an outlet line for the first reaction mixture,
b) a first separation unit comprising an inlet line for the first reaction mixture being connected with the outlet line for the first reaction mixture of the reactor a), an outlet line for a bromopropane enriched composition and an outlet line for a hydrogen bromide enriched composition,
c) a reactor for catalytically reacting the bromopropane enriched composition so as to produce a second reaction mixture containing propylene and hydrogen bromide, wherein the reactor comprises an inlet line for the bromopropane enriched composition being connected with the outlet line for the for the bromopropane enriched composition of the separation unit b), and an outlet line for the second reaction mixture,
d) a second separation unit comprising an inlet line for the second reaction mixture being connected with the outlet line for the second reaction mixture of the reactor c), an outlet line for propylene and an outlet line for a hydrogen bromide enriched composition, and
e) an electrolysis cell comprising an anode, a cathode and a membrane sandwiched between the anode and the cathode as well as an inlet line for a hydrogen bromide containing composition, an outlet line for hydrogen and an outlet line for a bromine containing composition, wherein the inlet line of the electrolysis cell is connected with the outlet line for the hydrogen bromide enriched composition of the first separation unit and/or with the outlet line for the hydrogen bromide enriched composition of the second separation unit.

13. The plant in accordance with claim 12, wherein the first separation unit comprises:
- a first distillation column comprising an inlet line being connected with the outlet line for the first reaction mixture of the reactor a), an overheads outlet line and a bottom outlet line,
- an absorption column comprising an inlet line being connected with the overheads outlet line of the first distillation column, an inlet line for absorbing agent, an outlet line for a hydrogen bromide enriched composition and an outlet line for a purified propane composition and
- a second distillation column comprising an inlet line being connected with the bottom outlet line of the first distillation column, an overheads outlet line for the bromopropane enriched composition and a bottom outlet line.

14. The plant in accordance with claims 12 or 13, wherein the second separation unit comprises:
- a first distillation column comprising an inlet line being connected with the outlet line for the second reaction mixture of the reactor c), an overheads outlet line and a bottom outlet line,
- an absorption column comprising an inlet line being connected with the overheads outlet line of the first distillation column, an inlet line for absorbing agent, an outlet line for a hydrogen bromide enriched composition and an outlet line for propylene or for a composition comprising propylene and hydrogen and
- a second distillation column comprising an inlet line being connected with the bottom outlet line of the first distillation column, an overheads outlet line and a bottom outlet line.

15. The plant in accordance with any of claims 12 to 14, which further comprises a purification unit, wherein the purification unit comprises a distillation column comprises an inlet line being connected with the outlet line for the bromine containing composition of the electrolysis cell e), an overheads outlet line and a bottom outlet line, wherein the purification unit further comprises a dryer comprising an inlet line being connected with the overheads outlet line of the distillation column and an outlet line for purified bromine, which is connected with the inlet line of the reactor a).
